# EUROPEAN PATENT SPECIFICATION

(11) **EP 1 256 805 B1**
(45) Date of publication and mention of the grant of the patent: **22.02.2006**
(21) Application number: 02009306.8
(22) Date of filing: 30.04.2002
(51) Int. Cl.: G01N 33/543

(54) **Biological material chip**
Chip aus biologischen Materialien
Puce de matériaux biologiques

(30) Priority: 09.05.2001 JP 2001138496
(43) Date of publication of application: 13.11.2002
(73) Proprietor: FUJI PHOTO FILM CO., LTD., Minamiashigara-shi, Kanagawa-ken 250-0193 (JP)
(72) Inventor: Inomata, Hiroko, Asaka-shi, Saitama 351-8585 (JP); Kojima, Masayoshi, Asaka-shi, Saitama 351-8585 (JP); Sudo, Yukio, Asaka-shi, Saitama 351-8585 (JP); Shinoki, Hiroshi, Asaka-shi, Saitama 351-8585 (JP); Iwaki, Yoshihide, Asaka-shi, Saitama 351-8585 (JP); Seshimoto, Osamu, Asaka-shi, Saitama 351-8585 (JP)
(74) Representative: Hiebl, Inge Elisabeth

(56) References cited:
- EP-A- 0 323 692
- EP-A- 0 456 304
- EP-A- 1 077 264
- EP-A- 1 106 603
- MACBEATH G ET AL: "Printing proteins as microarrays for high-throughput function determination" SCIENCE, vol. 289, 8 September 2000 (2000-09-08), pages 1760-1763, XP002190973 ISSN: 0036-8075

## Description

### FIELD OF THE INVENTION

The present invention relates to a biological chip such as a protein chip in which biological material such as DNA or protein is fixed to solid phase surface, which is useful for proteomics analysis, a method for the production of the chip, and a method for the detection of a target substance using the chip.

### BACKGROUND OF THE INVENTION

The technological development for efficiently analyzing gene functions of a variety of organisms has been rapidly progressed, and a detection tool called as a DNA chip where nucleotide derivatives such as a large number of DNA fragments or synthesized oligonucleotides or the like are fixed on the surface of a solid phase substrate has been employed in order to analyze the base sequence of the DNAs or DNA fragments. A molecule for detection such as a DNA or its fragments or a synthesized oligonucleotide like a nucleotide derivative bound to the surface of such a solid phase substrate is also referred to as a probe molecule. A representative DNA chip is a microarray in which a large number of probe molecules are aligned and fixed to a solid support such as a slide glass or the like. DNA chip related technologies relating to manufacturing of a DNA chip and its use are considered to be capable of also utilizable for detecting a biomolecules other than DNA. Therefore, these are expected to provide a new means for aiming at drug discovery research, development of a method of diagnosis of diseases or preventing the disease, or the like.

The DNA chip related technologies has been materialized since the fact that the method for determining the base sequence of a DNA by hybridization with an oligonucleotide was developed. Although this method could overcome the limitation of the method for determining the base sequence using gel electrophoresis, initially it did not come into practice.

Subsequently, by developing the DNA chip configured as described above and its preparation technique, the expression, mutation, polymorphism or the like of a gene has been capable of efficiently being examined in a short period of time. Specifically, a DNA fragment sample showing the complementarity to a DNA fragment or an oligonucleotide on the DNA chip prepared (which is also referred to as a target DNA fragment) is, in general, detected by utilizing the hybridization of the DNA fragment or the oligonucleotide on the DNA chip with the labeled DNA fragment sample.

In order to put the DNA chip preparation technique into practical use, a technology for aligning a large number of DNA fragments and oligonucleotides to surface of a solid support in high density and stable state is required.

As for a method for preparing a DNA chip, there are known a method for directly synthesizing oligonucleotide on the surface of the solid support (referred to as "on-chip method") and a method in which a DNA fragment or an oligonucleotide previously prepared is bound to the surface of the solid support. As for the on-chip method, a method for selectively synthesizing an oligonucleotide in the predetermined minute matrix region by combining use of a protective group selectively removable using photoirradiation, the photolithography technology used for fabricating a semiconductor device and a technology for synthesizing a solid phase (referred to as "masking technology") is representative.

As a method for binding and fixing a DNA fragment or an oligonucleotide previously prepared on the surface of the solid support, following methods are known corresponding to kinds of DNA fragments and kinds of solid supports.
(1) In the case where a DNA fragment to be fixed is a cDNA (complementary DNA synthesized by utilizing a mRNA as a template) or PCR products (DNA fragment obtained by amplifying the cDNA by a PCR method), in general, the cDNAs or PCR products are dotted to the surface of the solid support which was surface-treated with a polycationic compound (poly-lysine, polyethyleneimine or the like) using a spotter device provided in a DNA chip preparation device, and are electrostatically bound to the solid support by utilizing the charge held in the DNA fragments. As a method for treating the surface of the solid support, a method of employing a silane coupling agent containing an amino group, an aldehyde group, an epoxy group or the like is also utilized. In the surface treatment using the silane coupling agent, since the amino group, the aldehyde group or the like is fixed on the surface of the solid support by covalent bond, it is more stably fixed on the surface of the solid support, as compared with the case of surface treatment with a polycationic compound.
   As a modified method for utilizing the charge of the DNA fragments described above, there is reported a method wherein PCR products modified with an amino group is suspended in SSC (standard salt-citric acid buffer solution), dotted to the surface of the sililated slide glass, incubated, and then treated with sodium boron hydride and then with heating. However, there is a problem that it is difficult to necessarily obtain the sufficient fixation stability of the DNA fragments by this fixation method. In DNA chip technologies, detection limit is important. Therefore, binding and fixing of DNA fragments on the surface of the solid support in a sufficient amount (i.e., in a high density) and in a stable state has a direct influence on increase of the detection limit of hybridization of DNA fragment probes and labeled nucleic acid fragments samples.
(2) In the case where an oligonucleotide (probe molecule) to be fixed is a synthesized oligonucleotide, there is known a method, in which an oligonucleotide into which a reactive group has been introduced is synthesized, then the oligonucleotide is dotted to the surface of the solid support surface-treated so as to previously form the reactive group, thereby binding and fixing the oligonucleotide to the surface of the solid support by covalent bond. For example, there are known a method in which an amino group-introduced oligonucleotide is reacted with the surface of the slide glass to which an amino group is introduced in the presence of PDC (p-phenylene diisothiocyanate), and a method in which an aldehyde group- introduced oligonucleotide is reacted with said slide glass. These two methods are more advantageous from the viewpoint that an oligonucleotide is stably bound and fixed to the surface of the solid support, as compared with the above-described method (1) for electrostatically binding by utilizing the charge of the DNA fragments. However, there are problems that in a method of performing the reaction in the presence of PDC, the reaction of PDC and an amino group-introduced oligonucleotide is slow, and in a method of using an aldehyde group-introduced oligonucleotide, the stability of Schiff base which is a reactive product is low (that is, hydrolysis is easily occurred).

In recent years, a technology employing an oligonucleotide analog which is referred to as PNA (peptide nucleic acid) instead of an oligonucleotide or a polynucleotide (also including a synthesized oligonucleotide or polynucleotide and a DNA molecule and DNA fragment, and a RNA molecule and RNA fragment) as a probe molecule of a DNA chip has also been proposed. As a method for fixing PNA to the solid phase substrate by covalent bond, a method of using the combination of avidin and biotin is also known (Japanese Unexamined Patent Publication No.H11-332595 gazette). In this publication gazette, a technology utilizing a surface plasmon resonance (SPR) biosenser as the solid phase substrate is also described. Utilizing the DNA chip in which a probe molecule is fixed on the surface plasmon resonance biosenser, a DNA fragment bound to its surface via hybridization can be detected by utilizing the surface plasmon resonance phenomenon.

Moreover, as a substrate of the DNA chip, use of a charge coupled device (CCD) is also known (Nucleic Acid Research, 1994, Vol. 22, No. 11, pp. 2124-2125).

In Japanese Unexamined Patent Publication No. H04-228076 gazette (corresponding to U. S. Patent No. 5,387,505), a technology for isolating a target DNA is describe. In this technology, the target DNA having biotin molecule is bound to a substrate, the surface of which an avidins molecule is fixed on.

Japanese Patent Publication No.H07-43380 gazette (corresponding to U. S. Patent No.5, 094, 962) describes a detection tool used for ligand-receptor assay, that is, an analyzing tool that an receptor molecule is bound to surface of a microporous polymer particle having a reactively active group on its surface.

On the other hand, in recent years, since the genomic analysis has been almost completed, the "proteome/proteomics" research, that provides essential information in order to finally understand meanings of the gene information and to simulate the life-activities of the cells, has been progressed. The term "proteome" means the all sets of proteins which are translated and produced in a specific cell, an apparatus and an organ, and the research field of high-level information analysis of chemical structure, total amount, expression period, modification after translation, formation of aggregation and the like are referred to as "proteomics" .

The proteome research includes a profiling of proteins, an identification and precise analysis of proteins, a interaction network analysis and construction of a proteome data base, and is a field where these technologies are applied to the life science researches.

Among these, as the interaction network analysis method, a yeast two-hybrid method and a phage display method have been performed, and as a method of utilizing affinity capture, an immunoprecipitation method, a BIA-MA method, a column switching-mass spectrometry method and the like have been performed ("Proteome analysis method", pp. 163-211, published by Yodosha, Co., Ltd. ,2000) . However, any of these interaction network analyses listed above has not achieved a high throughput analysis.

The report has been made by Schreiber et al. on a protein microarray for a high throughput analysis of interaction of proteins (Science 289: 1760-1763, 2000). This is a method in which protein aqueous solution is dotted to the slide glass having an aldehyde group, blocked with BSA solution, then reacted with the protein solution to carry out detection by a fluorescence scanner. In this case, there is a problem that the stability of Schiff base which is a reaction product between an aldehyde group and an amino group is low (usually, hydrolysis is easily occurred).

In addition to these, as a method of fixing protein to the solid phase, a method, in which a hydrophobic polypeptide is introduced into the end of the protein, is described in Japanese Patent Publication No.H07-53108 gazette.

In Japanese Patent No.2, 922, 040, a method of fixing an antibody protein with protein A molecule film is described.

Besides, European patent application 0 323 692 discloses a reagent composed of:
(a) a polymeric particle covalently attached through reactive pendant groups to
(b) an immunological species which is capable of participating in an immunological reaction with a corresponding receptor,

the reagent characterized wherein the polymer is derived from at least one ethylenically unsaturated polymerizable monomer having either pendant activated 2-substituted ethylsulfonyl or vinylsulfonyl groups,
and
the interior of the particle being substantially free of detectable tracer material.

### SUMMARY OF THE INVENTION

An object of the present invention is to provide a biological material chip in which at least one member of specific binding partners is bound and fixed to a reactive solid support which can achieve rapid and stable binding and fixing. Another object of the present invention is to provide a method for the detection of a target substance which is another member of the specific binding partners, using the aforementioned biological material chip. Still another object of the present invention is to provide a method for the production of the aforementioned biological material chip.

In order to achieve the above objects, the inventors have earnestly studied and have prepared a biological material chip in which at least one member of specific binding partners was bound to a solid support by a covalent bond via sulfonyl group. As a result, they found that said member of specific binding partners can be promptly and stably bound to the solid support and a target material can be efficiently detected. The present invention has been accomplished on the basis of these findings.

According to the first aspect of the present invention, there is provided a biological material chip wherein a group represented by following formula (I) which contains a residue of a member of specific binding partners is bound to a solid support, wherein the formula is

-L-SO2-X-A (I)

and in the formula (I), L represents a linking group which binds -SO2-X-A and the solid support; X represents -CR1(R2)-CR3(R4)-; each of R1, R2, R3 and R4 represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and A represents a residue of the member of specific binding partners, said specific binding partners being a combination of an antibody or its fragment and a ligand, a combination of an antibody or its fragment and an antigen, a combination of an antibody or its fragment and a hapten, a combination of avidins and biotins or a combination of a receptor and a ligand and said solid support being glass, an electrode surface or a sensor chip surface.

According to the second aspect of the present invention, there is provided a method for the detection of a target substance in a specimen comprising the steps of:
(a) contacting a biological material chip wherein a group represented by following formula (I) containing a residue of a member of specific binding partners is bound to a solid support with a specimen containing a target substance which is another member of the specific binding partners; and
(b) analyzing interaction between said members of specific binding partners, wherein the formula is

   -L-SO2-X-A (I)

and in the formula (I), L represents a linking group which binds -SO2-X-A and the solid support; X represents -CR1(R2)-CR3(R4)-; each of R1, R2, R3 and R4 represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and A represents a residue of one member of specific binding partners, said specific binding partners being a combination of an antibody or its fragment and a ligand, a combination of an antibody or its fragment and an antigen, a combination of an antibody or its fragment and a hapten, a combination of avidins and biotins or a combination of a receptor and a ligand and said solid support being glass, an electrode surface or a sensor chip surface.

According to the third aspect of the present invention, there is provided a method for the production of a biological material chip as claimed in claim 1, comprising a step of contacting at least one member of specific binding partners containing a reactive group which forms a covalent bond by reacting with a vinylsulfonyl group or its reactive precursor group represented by following formula (II), with a solid support having the vinylsulfonyl group or its reactive precursor group represented by following formula (II) on its surface, wherein the formula is

-L-SO2-X' (II)

and in the formula (II), L represents a linking group which binds -SO2-X' and the solid support; X' represents -CR1=CR2 (R3) or -CH(R1)-CR2(R3)(Y); each of R1, R2 and R3 represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; Y represents a group which is substituted by a nucleophilic reagent or a group which is eliminated as [HA] by a base, said specific binding partners being a combination of an antibody or its fragment and a ligand, a combination of an antibody or its fragment and an antigen, a combination of an antibody or its fragment and a hapten, a combination of avidins and biotins or a combination of a receptor and a ligand and said solid support being glass, an electrode surface or a sensor chip surface.

In connection with the biological material chip, the method for the detection of a target substance in a specimen, and the method for the production of the aforementioned biological material chip as mentioned above, the preferred embodiments are mentioned below.

The specific binding partners are consisted of constituent members which can form a biological specific binding.

The specific binding partners are a combination of an antibody or its fragment and a ligand, a combination of an antibody or its fragment and an antigen, a combination of an antibody or its fragment and a hapten, a combination of avidins and biotins, or a combination of a receptor and a ligand.

The avidins are avidin, streptavidin, or their altered bodies which can form a stable complex with biotin.

The biotins are biotin, biocytin, desthiobiotin, oxybiotin, or their derivatives which can form a stable complex with avidin.

Referential Examples of the specific binding partners are a combination of a nucleic acid and a nucleic acid or a combination of a nucleic acid and a nucleic acid binding substance.

The nucleic acid is a nucleotide derivative, a peptide nucleonic acid or an LNA.

The nucleic acid binding substance is a double stranded DNA recognition substance.

The double stranded DNA recognition substance is a double stranded DNA recognition antibody.

The double stranded DNA recognition substance is a DNA transcription factor.

The double stranded DNA recognition substance is a protein having a Zinc finger motif or a Ring finger motif.

The double stranded DNA recognition substance is a peptide nucleic acid.

In the formula (I), the "A" represents a residue of a protein.

The solid support is glass, an electrode surface or a sensor chip surface; and

A blocking treatment of a free reactive group on surface of said solid support is performed with an aqueous solution of an amino acid, a peptide or a protein, after contacting at least one member of specific binding partners with said solid support.

### BRIEF DESCRIPTION OF THE DRAWING

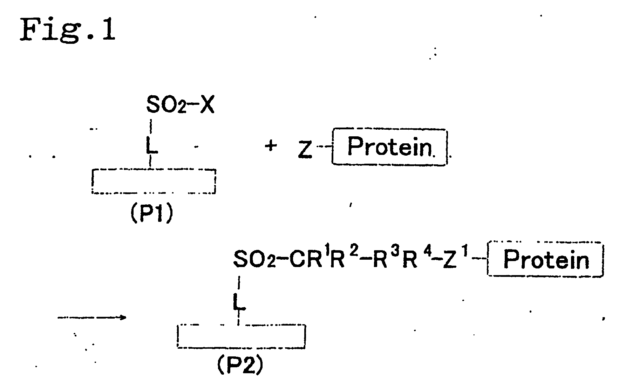

Fig. 1 is a schematic diagram showing the structure of a protein chip which is a typical embodiment of the present invention.

### DETAILED DESCRIPTION OF THE INVENTION

The present invention relates to a biological material chip wherein a group represented by following formula (I) which contains a residue of a member of specific binding partners is bound to a solid support.

-L-SO₂-X-A (I)

in the formula (I), L represents a liking group which binds -SO₂-X-A and the solid support; X represents -CR¹(R²)-CR³(R⁴)-; each of R¹, R², R³ and R⁴ represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; A represents a residue of the member of specific binding partners.

The specific binding partners in the present invention mean binding partners which form a biological specific binding. Examples thereof include, but are not limited to, combinations such as an antibody or an antibody fragment/a ligand, an antibody or an antibody fragment/an antigen, an antibody or an antibody fragment/substance having an antigenic determinant such as a hapten, a receptor/a ligand and avidins/biotins. When the solid support having a fixed DNA which is produced is used as DNA chip, the specific binding partners are those which have certain binding strength and can be used accurately and repeatedly in the subsequent hybridization process.

Referential examples of biotins include biotin, biocytin, desthiobiotin, oxybiotin, or their derivatives that can form a stable complex with avidin. The phrase "can form a stable complex" means that a complex having a dissociation constant approximate to the dissociation constant of biotin-avidin complex (10⁻¹⁵M) can be formed. Examples of avidins include avidin, streptavidin, or these altered bodies that can form a stable complex with biotin. The phrase "a stable complex" also means the same matter as defined above with respect to biotins. Further, the altered body means a modified body or its fragment of naturally occurring avidin or streptavidin, or recombinants thereof.

Referential Examples of the nucleic acid include, but are not limited to, nucleotide derivatives or their analogs. Representative examples include an oligonucleotide, a polynucleotide, and a peptide nucleic acid. The nucleotide derivative or its analog may be naturally occurring one (DNA, DNA fragment, RNA or RNA fragment and the like), or may be a synthetic compound. Moreover, nucleotide derivatives or their analogs include a variety of analogous compounds such as what is called an LNA having a crosslinking group at its sugar unit portion (J. Am. Chem. Soc. 1998, 120:13252-13253).

The nucleic acid binding substances include a double stranded DNA recognition substance, but are not limited thereto. The double stranded DNA recognition substances include a substance which recognizes a double stranded DNA and specifically binds to it. Examples of the double stranded DNA recognition substance include a DNA transcription factor, a mismatch repairing protein, a double stranded DNA recognition antibody, or a peptide nucleic acid. Furthermore, the double stranded DNA recognition substances include substances having Zinc Finger motif or Ring finger motif.

The DNA transcription factor is a substance that binds to the promoter region on gene and controls the transcription from DNA to mRNA (Takaaki, Tamura: Transcription Factor, published by Yodosha, Co., Ltd., 1995). Accordingly, it is known that the transcription factor specifically binds to a double stranded DNA of the specific sequence.

Among a large number of transcription factors, Zinc Finger Protein, that is, a transcription factor group having Zinc finger and Ring Finger motifs, shows a very high occurrence rate in eucaryote, and 1% of the genome seems to code for them. Plabo et al. have analyzed the tertiary structure of Zinc Finger motif and elucidated the mechanism of its binding to DNA (Science 252:809 (1991)). Further, Choo et al. have succeeded in preparing a Zinc Finger Protein group which binds to the specific sequence but which does not exist in the nature, by a gene recombinant method (Nature 372: 642 (1994), PNAS 91: 11163 (1994)). Furthermore, the Scripps Research Institute group has succeeded in preparing a novel Zinc Finger Protein group by Phage Display (PNAS 95: 2812 (1998); 96: 2758 (1999)). As described above, the DNA transcription factor group represented by Zinc Finger Protein originally has the nature of binding to a double stranded DNA, and according to the researches in recent years, it has been made possible to prepare a recombinant which recognizes a given DNA sequence. It is possible to efficiently capture a double stranded DNA on a support by fixing such proteins.

Besides these, the nucleic acid binding substances include a helix-loop-helix protein and a substance having an Ets domain.

In the case where the member of specific binding partners to be fixed to the solid support is proteins, a group such as an amino group, an imino group, a hydrazine group, a carbomoyl group, a hydrazinocarbonyl group, a mercapto group, or a carboxyimido group may be introduced to said proteins. By using an amino group or an mercapto group present in the protein, or the introduced group mentioned above, a covalent bond can be formed with a reactive group via a sulfonyl group.

The solid support to which the aforementioned member of specific binding partners (for example, antibody, avidins or nucleic acid binding substance) have been fixed, can fix another member (e.g., ligand, biotins, or nucleic acid) of binding partners which is capable of specifically reacting with said fixed members by contacting the solid support with said another member of the specific binding partners under the presence of an aqueous medium. It is desirable that a detectable label (e.g., fluorescence label, enzyme label or the like) is bound to another member of the specific binding partners to be fixed (e.g., ligand, biotins, or nucleic acid) in such a way that the fixation can be detected from the exterior.

Comparative Examples of a nucleotide derivative or its analog in the case where the substance to be fixed to the solid support is a nucleic acid, include an oligonucleotide, a polynucleotide, a peptide nucleic acid, or an LNA. These nucleotide derivatives or their analogs include compounds that have a reactive group at or nearby one of end portions of a molecule, which can react with a vinylsulfonyl group or its reactive precursor group to form a covalent bond are utilized. Examples of such reactive group include an amino group, an imino group, a hydrazino group, a carbamoyl group, a hydrazinocarbonyl group, a carboxyimido group, a mercapto group and the like.

The solid support to which the aforementioned nucleotides derivative or their analogs are fixed, can fix an oligonucleotide or a polynucleotide (DNA or its fragment, or RNA or its fragment) that shows complementarity to one of said fixed nucleotide derivatives or their analogs by contacting the solid support with the complementary oligonucleotide or polynucleotide to perform hybridization. It is desirable that a detectable label (e.g., fluorescence label) is bound to the complementary oligonucleotide or polynucleotide to be fixed in such a way that the fixation can be detected from the exterior.

The solid support used in the present invention may be any form including, for example, a plate, a microwell, beads, a stick or the like, so long as it does not have an adverse effect on binding formation between respective members of the specific binding partners. It is preferred to use a substrate having the surface with properties of, especially hydrophobic or low hydrophilic nature and smoothness. Further, a substrate that has a surface of low smoothness with a convex and a concave can be used. The substance for the solid support include glass, cement, ceramics or new ceramics such as potteries; polymers such as polyethylene terephthalate, cellulose acetate, polycarbonate of bisphenol A, polystyrene, or polymethyl methacrylate; silicon; a variety of porous substances such as activated carbon, porous glass, porous ceramics, porous silicon, porous activated carbon, woven fabric, knitted fabric, non-woven fabric, filter paper, short fiber, or membrane filter. It is preferable that the size of a fine hole of a porous substance is in the range from 2 to 1000 nm, and particularly preferable in the range from 2 to 500 nm. It is particularly preferable that the substance quality of the solid support is glass or silicon. This is because of the easiness of surface treatment and the easiness of analysis by an electrochemical method. It is preferable that the thickness of the solid support is in the range from 100 to 2000 *µ*m. The solid support may be processed in a form of magnetic material or an electrode for the convenience of operation.

The solid support may be an electrode substrate which is used as a substrate of a DNA chip used for an electrochemical analyzing method. Further, a variety of functional substrates such as a substrate used for the above-described surface plasmon resonance (SPR) biosensor, or a charge coupled device (CCD), may also be used.

In the present invention, a member of a specific binding partners (in the following formula (I), a residue represented by A) of the biological material chip is bound to the solid support by a covalent bond via a sulfonyl group, as is shown in the following formula (I).

-L-SO₂-X-A (I)

in the formula (I), L represents a liking group which binds -SO₂-X-A and the solid support; X represents -CR¹(R²) -CR³(R⁴) -; each of R¹, R², R³ and R⁴represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and A represents a residue of the member of specific binding partners.

In the formula (I), L represents a bivalent or more linking group for binding -SO₂-X-A and the solid support. Examples of a -L- include any linking group selected from aliphatic, aromatic or heterocyclic compounds, hydro carbon chains that may be interrupted by a hetero atom, or combinations of them. Furthermore, L may be a singl bond.

In the formula (I), examples of an alkyl group having 1 to 6 carbon atoms include a methyl group, an ethyl group, a n-propyl group, a n-butyl group, and a n-hexyl group, and the methyl group is preferable. Examples of an aryl group having 6 to 20 carbon atoms include a phenyl group and a naphthyl group. It is preferable that each of R1, R2 and R3 represents a hydrogen atom.

Examples of an aralkyl group having 7 to 26 carbon atoms in total containing an alkyl chain having 1 to 6 carbon atoms include the combination of the examples of an alkyl group having 1 to 6 carbon atoms and the examples of an aryl group having 6 to 20 carbon atoms.

Furthermore, the present invention relates to a method for the detection of a target substance in a specimen comprising the steps of:
(a) contacting a biological material chip wherein a group represented by the aforementioned formula (I) containing a residue of a member of specific binding partners is bound to a solid support with a specimen containing a target substance which is another member of the specific binding partners; and
(b) analyzing interaction between said members of specific binding partners.

The type of "specimen containing a target substance" used in the present invention is not particularly limited, and includes, for example, a blood such as peripheral venous blood, white blood cell, serum, urine, stool, sperm, saliva, a cultured cell, a tissue cell such as a variety of cells of organs, and any other sample containing nucleic acid. As for the specimen, the sample such as the tissue cell as described above may be used as it is. However, preferably, nucleic acid, ligand or the like which has been released by destructing the cell in the specimen is used as a specimen. The destruction of the cell in the specimen can be performed according to the conventional manner. For example, it can be performed by adding a physical action such as shaking, supersonic treatment from the exterior. The nucleic acid also can be released from the cell using a nucleic acid extraction solution (e.g., solution containing a surfactant such as SDS, Triton-X, Tween-20 or the like, or saponin, EDTA, protease or the like, or the like). In the case where the nucleic acid is eluted using a nucleic acid extraction solution, the reaction can be promoted by incubation at the temperature of 37°C or higher.

Furthermore, the present invention relates to a method for producing the chip according to the present invention comprising the step of contacting the solid support which contains a vinyl sulfonyl group or its reactive precursor group represented by the following formula (II) on the surface, with at least one member of specific binding partners having a reactive group which forms a covalent bond by reacting with the vinyl sulfonyl group or its reactive precursor group.

-L-SO₂-X' (II)

in the formula (II), L represents a linking group for binding -SO₂-X' and the solid support; X' represents -CR¹=CR² (R³) or -CH(R¹)-CR²(R³) (Y) ; each of R¹, R² and R³ represents independently from each other, a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and Y represents a group substituted by a nucleophilic reagent, or a group which is eliminated as "HY" by base.

In the formula (II), examples of an alkyl group having 1 to 6 carbon atoms include a methyl group, an ethyl group, a n-propyl group, a n-butyl group, and a n-hexyl group, and the methyl group is particularly preferable. Examples of an aryl group having 6 to 20 carbon atoms include a phenyl group and naphthyl group. It is preferable that each of R¹, R² and R³ represents a hydrogen atom.

Examples of an aralkyl group having 7 to 26 carbon atoms in total containing an alkyl chain having 1 to 6 carbon atoms include the combination of the examples of an alkyl group having 1 to 6 carbon atoms and the examples of an aryl group having 6 to 20 carbon atoms.

In the formula (II), Y represents a group substituted by a nucleophilic reagent such as -OH, -OR⁰, -SH, NH₃, NH₂R⁰ (R⁰ represents a group such as alkyl group or the like except for hydrogen atom), or a group which is eliminated as "HY" by base. Examples thereof include a halogen atom, -OSO₂R¹¹, -OCOR¹², -OSO₃M, or a quaternary pyridinium group (R¹¹ represents an alkyl group having 1 to 6 carbon atoms, an aryl group having 6 to 20 carbon atoms, or an aralkyl group having 7 to 26 carbon atoms in total containing an alkyl chain having 1 to 6 carbon atoms; R¹² represents an alkyl group having 1 to 6 carbon atoms or halogenated alkyl group having 1 to 6 carbon atoms; M represents a hydrogen atom, an alkali metal atom, or an ammonium group) .

An alkyl group of R¹¹, an aryl group of R¹¹ and an aralkyl group of R¹¹ may have a substituent. Examples of such a substituent include an atom or a group selected from the group consisted of a hydroxyl group, an alkoxy group having 1 to 6 carbon atoms, an alkenyl group having 1 to 6 carbon atoms, a carbamoyl group having 2 to 7 carbon atoms, an alkyl group having 1 to 6 carbon atoms, an aralkyl group having 7 to 16 carbon atoms, an aryl group having 6 to 20 carbon atoms, an sulfamoyl group (or its sodium salt, potassium salt or the like), a sulfo group (or its sodium salt, potassium salt or the like), a carboxylic acid group (or its sodium salt, potassium salt or the like), a halogen atom, an alkenylene group having 1 to 6 carbon atoms, an arylene group having 6 to 20 carbon atoms, sulfonyl group and their combinations.

In the formula (II), L represents a bivalent or more linking group for linking -SO₂-X' group and the solid support. Examples of a -L- include any linking group selected from aliphatic, aromatic or heterocyclic compound and a hydrocarbon chain that may be interrupted by a hetero atom, and a linking group selected from their combinations. Further L may be a single bond.

Preferable examples of the above-described "-X "' group are shown thereinafter:

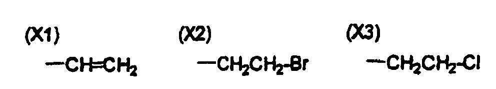

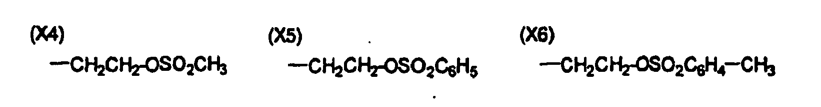

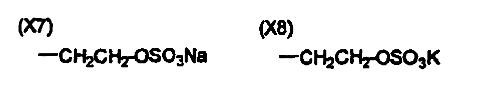

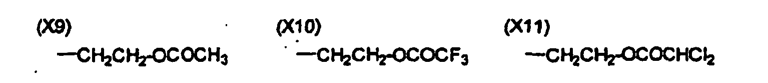

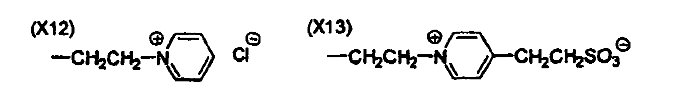

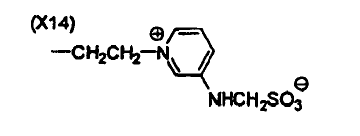

In the above-described concrete examples, it is preferable that "-X' " represents (X1), (X2), (X3), (X4), (X7), (X8), (X13) or (X14), and more preferable that "-X' " represents (X1) or (X2). And it is particularly preferable that "-X' " represents a vinyl group represented by (X1).

Since a covalent bond via a sulfonyl group utilized in the present invention has a high resistance to hydrolysis, it can be readily stored in a stable state, and can rapidly react with a reactive group of nucleotide derivatives or their analogs which previously contain an amino group or to which a reactive group such as an amino group has been introduced, to form a stable covalent bond.

On one end of a nucleotide derivative or its analog such as an oligonucleotide and a DNA fragment, a reactive group which forms a covalent bond by reacting with the aforementioned vinylsulfonyl group or its reactive precursor group is introduced. Preferred examples of such a reactive group include an amino group, an imino group, a hydrazino group, a carbomoyl group, a hydrazinocarbonyl group, a carboxyimido group or a mercapto group, and the amino group is particularly preferable. The reactive group is usually bound to an oligonucleotide and a DNA fragment via a crosslinker. As the crosslinker, for example, an alkylene group or an n-alkylamino-alkylene group is utilized, and a hexylene group or an n-methylamino-hexylene group is preferable, and a hexylene group is particularly preferable. It should be noted that since a peptide nucleic acid (PNA) has an amino group, usually it is not necessary to introduce another reactive group.

Likewise, since the protein has an amino group or a mercapto group, usually it is not necessary to introduce another reactive group. However, since the tertiary structure of the protein largely relates to its function, in the case where the activity of the protein is lowered, it is preferable to introduce a reactive group at a specific position having no influence on the activity.

Contact of a nucleotide derivative or its analog having a reactive group with a reactive solid support is usually carried out by dotting the aqueous solution of the nucleotide derivative or its analog to the surface of reactive solid support. Concretely, it is preferable that an aqueous liquid is prepared by dissolving or dispersing the nucleotide derivative or its analog having a reactive group in an aqueous medium, and then the aqueous liquid is poured into 96 well or 384 well plastic plate, and the poured aqueous liquid is dropped to the surface of the solid support using a spotter device or the like.

In the case of dotting of the protein, a spotter device can be utilized for dotting of an aqueous liquid. However, there is a possibility of lowering the activity of the protein depending on the property of a pin-head. In this case, it may be more preferable to use an ink jet device or the like.

In order to prevent the nucleotide derivative or its analog from being dried after the dotting, a substance having a high boiling point may be added in the aqueous liquid in which the nucleotide derivative or its analog is dissolved or dispersed. The substance having a high boiling point is preferably a substance that can be dissolved in the aqueous solution in which the nucleotide derivative or its analog to be dotted is dissolved or dispersed, does not hinder hybridization with a sample such as a nucleic acid fragment sample (target nucleic acid fragment) which is an object of the detection, and has a not very high viscosity. Such substances include glycerin, ethylene glycol, dimethyl sulfoxide and a hydrophilic polymer having a lower molecular weight. Examples of the hydrophilic polymers include polyacrylamide, polyethylene glycol and sodium polyacrylate. Preferable molecular weight of this polymer is in the range from 10³ to 10⁶. As the substance having a high boiling point, it is more preferable to use glycerin or ethylene glycol, and particularly preferable to use glycerin. Preferable concentration of the substance having a high boiling point is in the range from 0.1 to 2% by volume, and particularly preferably 0.5 to 1% by volume in the aqueous liquid of the nucleotide derivative or its analog.

In order to prevent the protein from being dried and denatured after dotting, the substance having a high boiling point may be added in the aqueous liquid as is the case with the nucleotide derivative or its analog. There is no regulation on concentration of the substance having a high boiling point in an aqueous liquid of the nucleotide derivative or its analog. The concentration may be adjusted depending on an activity of a protein after dotting.

Moreover, for the sake of the same purpose, it is also preferable to place the solid support after dotting of the nucleotide derivatives or their analogs, proteins or the like under the circumstances where the humidity is 90% or more and the temperature is in the range from 25 to 50°C (in the case of the protein, up to 37°C).

A preferable fixed amount (numerical quantity) of the protein, nucleotide derivative or its analogue on the surface of the solid support is in the range from 1 to 10⁵ kind/cm². By dotting, the aqueous liquid of the protein, the nucleotide derivative or its analog is fixed in a dot shape to the surface of the solid support. The shape of the dot is nearly circular. The distance between the respective dots, the size of the dot and the volume of the aqueous liquid when it is dotted, vary depending on its intended use.

In FIG. 1, a configuration of a protein chip, which is the representative embodiment of the present invention, is schematically shown.

When the protein A having the reactive group (Z) is dotted to the surface of the solid support (P1) shown in FIG. 1, the reaction between X and the protein occurs. However, an unreacted X to which the protein is not bound also exists on the surface of the solid support (P1). In this case, there is a possibility that such X reacts in a non-specific manner with a labeled ligand sample and the like in a reaction to be performed later, resulting in a problem that non-specific binding may be measured. Therefore, it is preferable that the X has been previously subjected to a blocking treatment. It is preferable that the blocking treatment is performed by bringing a compound having an amino group or a mercapto group into contact with the surface of the solid support (P2). In order to prevent the non-specifical binding of a ligand to be reacted, it is preferable to perform the blocking treatment using a protein blocking agent, specifically, BSA, casein, gelatin or the like. As the result of the blocking, BSA or the like exists on the surface of the solid support (P2) which has not been dotted, thereby preventing the binding of the ligand. Moreover, in the case where the nucleic acid is to be reacted, the blocking treatment can be carried out by contacting an anionic compound having an amino group or a mercapto group other than the above-described protein blocking agent. In the case where the substance with which the protein is reacted is a nucleic acid, since the nucleic acid has a negative charge, it can prevent the nucleic acid from reacting with an unreacted X by generating the negative charge also on the surface of the solid support (P2). As for such an anionic compound, any compound can be used if it reacts with X and has a negative charge (COO⁻, SO₃⁻, OSO₃⁻, PO₃⁻, or PO₂⁻) . Among them, an amino acid is preferable, and glycine or cysteine is particularly preferable. Further, taurine is also preferably used.

A protein chip which is a representative aspect of the present invention is utilized for the analysis of protein interactions, the analysis of the protein expression and the drug development search. Furthermore, in the case where the protein is a nucleic acid binding protein, it can be utilized for the mutation analysis and the nucleotide polymorphism analysis depending on its recognition nucleic acid sequence.

The principle of the detection is based on the reaction with the labeled ligand or nucleic acid. As labeling methods, although a RI method and a non-RI method (fluorescence method, biotin method, chemiluminescence method or the like) are known, it is not particularly limited. For example, in the case of the fluorescence method, as a fluorescent substance utilized for a fluorescence labeling, any can be used if it can bind to the basic portion of nucleic acid or protein amino acid residue. For example, cyanine dye (e.g., Cy3, Cy5 or the like of Cy Dye™ series, which is commercially available), rhodamine 6G reagent, N-acetoxy-N2-acetylaminofluorene (AAF) or AAIF (iodine derivative of AAF) can be used.

It is preferable that the target nucleic acid in the specimen is directly detected without amplifying it by a PCR method or the like. However, it may be detected after it has been previously amplified. The target nucleic acid or its amplified body can be easily detected by previously labeling it. In order to label the nucleic acid, a method of using an enzyme (Reverse Transcriptase, DNA polymerase, RNA polymerase, Terminal deoxy trasferase or the like) is often used. Further, the labeling substance may be directly bound by chemical reaction. Such labeling method has been described in some books as the known technology (Shintaro Nomura: De-isotope Experiment Protocol 1, published by Shujun Sha, Co., Ltd., 1994; Shintaro Nomura: De-isotope Experiment Protocol 2, published by Shujun Sha, Co., Ltd., 1998; Masaaki Muramatsu: DNA Microarray and Advanced PCR Method Labeling Material, published by Shujun Sha, Co., Ltd., 2000). It is preferable that the labeling material is a material capable of making a detectable signal. In the case where the labeling material is a material having an amplifying ability of signal such as an enzyme and a catalyst, the detection sensitivity of DNA is largely enhanced.

However, since the labeling operation described above is generally troublesome, a method of measuring the nucleic acid in the specimen without previous labeling of the nucleic acid can be used as a more preferable method of detection. For the purpose of it, for example, a DNA intercalating agent which recognizes a double stranded DNA, that is, what is called a DNA intercalator can be used. By the use of a DNA intercalator, not only the operation of the detection is made easier, but also the sensitivity of the detection is enhanced. For example, in the case where a DNA of 1000 bp is to be detected, although a labeling method can introduce several labeling materials at most, in the case where the intercalator is used, 100 or more labeling materials can be introduced.

The DNA intercalator may be a material which can form a detectable signal in itself, or the signal formation material may be bound to the side chain of intercalator, or bound to the intercalator via a specific binding pair such as a biotin-avidin, an antigen-antibody or a hapten-antibody. It is preferable that the detectable signal used in the present invention is the signal detectable by a fluorescene detection, a luminescence detection, a chemiluminescence detection, a bioluminescence detection, an electrochemiluminescence detection, a radiation detection, an electrochemical detection or a colorimetric detectsion, but it is not limited to them.

In the case where a ligand is a target, there can be used a substance obtained by reacting a succinimide body of a cyanine dye (e.g., Cy3, Cy5 or the like of Cy Dye™ series, which is commercially available), rhodamine 6G reagent, N-acetoxy-N₂-acetylaminofluorene (AAF) or AAIF (iodine derivative of AAF) with an amino group existing inside.

It is preferable that hybridization is carried out by dotting an aqueous solution, which is previously pipetted into a 96 wells or 384 wells plastic plate, in which labeled nucleic acid fragment samples are dissolved or dispersed, to the solid support of the present invention to which nucleotide derivatives or its analogs are fixed. The preferable amount of the solution for dotting is in the range from 1 to 100 nL. The hybridization is preferably carried out in the temperature range from room temperature to 70°C, and for the period from 1 to 20 hours. After the completion of hybridization, it is preferable that washing are performed using a mixed solution of a surfactant and a buffer solution to remove unreacted nucleic acid fragment samples. As a surfactant, it is preferable to use sodium dodecyl sulfate (SDS). As a buffer solution, citrate buffer solution, phosphate buffer solution, borate buffer solution, Tris buffer solution, Good' buffer solution or the like can be used. It is particularly preferable to use citrate buffer solution.

The hybridization using the solid support to which nucleotide derivatives or its analogs are fixed is characterized in that the amount of usage of the labeled nucleic acid fragment samples can be decreased to a very minute amount. Therefore, it is necessary to set the optimal conditions of the hybridization depending on the length of chain of the nucleotide derivatives or its analogs fixed to the solid support and the types of the labeled nucleic acid fragment samples. For the analysis of gene expression, it is preferable that a hybridization for a long time period is performed so as to be capable of sufficiently detecting even a lower expressing gene. For the detection of a single nucleotide polymorphism, it is preferable that a hybridization for a short period is performed. Moreover, it is also characterized in that comparison or quantitative determination of the expression amount are made possible using a single solid support to which DNA fragments are fixed by previously preparing two types of the nucleic acid fragment sample labeled by fluorescent substances different from each other and using them in a hybridization at the same time.

The present invention will be more concretely described below by the following examples. However, these examples are offered only to help an easy understanding of the present invention, and are not intended to limit the scope of the present invention.

### Examples

### Example 1: Detection of complementary target oligonucleotide sample (Referential Example)

### (1) Preparation of solid support to which vinylsulfonyl group has been introduced

After immersing a slide glass (25 mm x 75 mm) in an ethanol solution of aminopropylethoxy silane (2% by weight) (Shin-Etsu Chemical) at 110°C for 10 minutes, the slide glass was taken out of the solution. Then, the slide glass was washed by ethanol, and dried at 110°C for 10 minutes to prepare a silane compound-coated slide glass (A). Next, the silane compound-coated slide glass was immersed in a phosphate buffer solution (pH 8.5) of 1,2-bis (vinylsulfonylacetamide) ethane (5% by weight) for 1 hour, and was taken out of the solution. Then, the slide glass was washed with acetonitrile, and dried for 1 hour under a reduced pressure condition to obtain a solid support (B) where the vinylsulfonyl group was introduced to its surface.

### (2) Preparation of oligonucleotide-fixed solid support

An aqueous liquid (1 x 10⁻⁶M, 1µL) of dispersion of 40mer oligonucleotide fragment: (3'-TCCTCCATGTCCGGGGAGGATCTGACACTTCAAGGTCTAG-5') (Sequence No. 1) of which 3' end was modified with an amino group in 0.1M carbonate buffer solution (pH 9.3), was dotted to the solid support (B) obtained in the above (1) of Example 1. Immediately, the solid support after the dotting was left for 1 hour at the temperature of 25°C and the humidity of 90%. Then, the solid support was washed with the mixed solution of 0.1% by weight SDS (sodium dodecyl sulfate) and 2 x SSC (2 x SSC: solution obtained by diluting the stock solution of SSC by 2-fold; SSC: standard salt-citrate buffer solution) two times, and washed with 0.2 x SSC aqueous solution once. Then, the slide glass after the above washing was immersed in 0.1 M glycine aqueous solution (pH 10) for 1 hour and 30 minutes, washed with distilled water, and dried at room temperature to obtain a solid support (C) to which the oligonucleotides were fixed.

### (3) Detection of complementary target oligonucleotide sample

An aqueous dispersion liquid of the 22mer target oligonucleotide sample (3'-CTAGTCTGTGAAGTTCCAGATC-5') (Sequence No. 2) of which 5' end was bound with Cy5 (fluorescence label) in the hybridization solution (mixed solution of 4 x SSC and 10% by weight of SDS, 20µl), was dotted to the solid support (C) obtained in the above-described (1). Then, after protecting the surface with a cover glass for microscopy, the solid support was incubated at 60°C for 20 hours within a moisture chamber. After the incubation; the solid support was washed with a mixed solution of 0.1% by weight SDS and 2 x SSC, a mixed solution of 0.1% by weight SDS and 2 x SSC, and 0.2 x SSC aqueous solution in turn, centrifuged at 700 rpm for 5 minutes, and dried at room temperature. Fluorescence intensity of the surface of the slide glass measured by a fluorescence scanning device was 1219, and it was largely increased comparing with the background fluorescence intensity. Thus, it is understood that by using the oligonucleotide-fixed solid support prepared according to the fixation method of the present invention, a target oligonucleotide sample such as a target DNA fragment sample having the complementarity to the oligonucleotide fixed to the oligonucleotide-fixed solid support can be efficiently detected.

### Example 2: Detection of complementary cDNA using cDNA chip (Referential Example)

### (1) Preparation of amino-terminal DNA

An amino-terminal DNA (GP-NH2) for dotting was prepared by the PCR method using a 20 mer primer (5'-TGGCCGCCTTCAACGCTCAG-3') (Sequence No. 3) and a 24 mer primer (5'-GAAGGTGTGGCGCAGGTCGTAGTG-3') (Sequence No. 4) of which their 5' ends were bound with an amino group, and using pCR-ScriptTM SK (+)-α-2-HS-glycoprotein as a template. As for PCR conditions, reaction was performed for 30 cycles of 94°C/20 seconds, 60°C/30 seconds and 72°C/30 seconds using Pyrobest DNA Polymerase. PE Thermal Circular 9700 was used. Under the similar PCR conditions, an amino-terminal DNA (Act-NH2) for dotting was prepared using a 23 mer primer (5'-ATGGATGATGATATCGCCGCGCT-3') (Sequence No. 5) and a 24 mer primer (5'-GGTGAGGATCTTCATGAGGTAGTC-3') (Sequence No. 6) of which their 5' ends were bound with an amino group, and using pBlueScript II SK(+)-β-Actin as a template.

### (2) Preparation of DNA-fixed solid support

An aqueous liquid (1 x 10⁻⁶M, 1 *µ*L) of dispersion of GP-NH2 or Act-NH2 in 0.1M carbonate buffer solution (pH 9.3), was dotted by a spotter device to the solid support having vinylsulfonyl group on its surface obtained in Example 1 (1). Immediately, the solid support after the dotting was left at 25°C overnight within the saturated saline solution chamber. Then, the solid support was immersed in a 0.5M glycin aqueous solution (pH8.5) for 1 hour, and left in boiling water for 30 minutes to denature double strand into single strand. Then, the denaturing was terminated by immersing the solid support in an iced ethanol, and the solid support was dried at room temperature to obtain a solid support (D) to which the cDNAs were fixed.

### (3) Preparation of fluorescence dye-labeled cDNA target using reverse transcription reaction

A fluorescence dye-labeled cDNA target was prepared from a 22mer primer (5'-ACTGTGCGTGTTTTCCGGGGGT-3') (Sequence No. 7) by a reverse transcription reaction using Gp-cRNA, which was prepared by in vitro transcription, as a template. CRNA (2 *µ* g), primer (20pmol), dATP, dGTP and dCTP at the final concentration of 500 *µ*M, dTTP at the final concentration of 200 *µ*M and Cy5-dUTP (Amersham Pharmacia Biotech) at the final concentration of 100 *µ*M were mixed. Then the mixture was adjusted to 13 *µ*L by adding DEPC-dH₂O (Life Technologies). After incubation at 65°C for 5 minutes, the mixture was rapidly cooled on ice. 4 *µ*L of 5 x SuperScriptII Buffer (Life Technologies), 1 *µ*L of RnaseOUT (Life Technologies) and 2 *µ*L of 0.1M DTT were added thereto. After incubation at 42°C for 2-3 minutes, 1 *µ*L of SuperScriptII reverse transcriptase (Life Technologies) was added, and the mixture was then incubated at 42°C for 30 minutes. Further, 1 *µ*L of SuperScriptII reverse transcriptase (Life Technologies) was added, and the mixture was incubated at 42°C for 30 minutes. EDTA at the final concentration of 50 mM and NaOH at the final concentration of 0.2 M were added, and the mixture was incubated at 65°C for 15 minutes. After neutralization with 1M Tris-HCl (pH 7.5), the mixture was subjected to an agarose gel electrophoresis. The gel was scanned by.a fluorescence scanner (FLA2000; Fuji Photo Film Co., LTD) to identify the Cy5-labeled target (GP-Cy5).

### (4) Hybridization of complementary target cDNA

The dispersion of 1 x 10⁻⁸M target cDNA (GP-Cy5) in a hybridization solution (mixed solution of 4 x SSC and 10% by weight SDS, 20 µL) was dotted onto the solid support (D) obtained in the above (2). After protecting the surface with a cover glass for microscopy, the solid support was incubated at 60°C for 20 hours within a moisture chamber. Then, the solid support was washed with a mixed solution of 0.1% by weight SDS and 2 x SSC, a mixed solution of 0.1% by weight SDS and 0.2 x SSC, and 0.2 x SSC aqueous solution, centrifuged at 700 rpm for 5 minutes, and dried at room temperature. Then, fluorescence intensity of the surface of the slide glass was measured by a fluorescence scanning device. At the GP-NH2 spot it was 337,000, which was largely increased comparing with the background fluorescence intensity. Further, the fluorescence intensity at the Act-NH2 spot which was a negative control, was 39,000, indicating that the signal was significantly increased at the GP-NH2 spot. Therefore, it is understood that by using the DNA-fixed solid support prepared according to the present invention where DNA is fixed via sulfonyl group, a target cDNA sample such as a target cDNA fragment sample having the complementarity to DNA fixed to the DNA-fixed solid support can be efficiently detected.

### Example 3: Detection of ligand by antibody-fixed slide

### (1) Fixation of antibody

Goat Anti-Human IgG (Jackson ImmunoResearch) was diluted with PBS (100, 20, 4, 0.8, 0.16 ng/ µL), and 1 µL of this diluted IgG was dotted to the solid support (B) prepared in the above Example 1(1). Immediately, the solid support after the dotting was left at 25°C for 3 hours in a saturated common salt chamber, then was subjected to a blocking treatment by immersing it in 1% BSA/0.05% Tween 20-PBS (PBS-T) for 1 hour, to obtain an antibody: slide (E).

### (2) Reaction with ligand and detection

HybriWell (Grace Bio-Labs) was closely contacted with the antibody slide (E) prepared in the above (1). Human IgG-Cy5 (Jackson ImmunoResearch) was diluted with 1% BSA/PBS-T into 2 *µ*g/ml. After 100 *µ*L of the diluted solution was added within HybriWell, it was incubated at 25°C for 1 hour within a moisture chamber. Subsequently, it was washed with PBS-T three times, rinsed with PBS, and dried by a centrifuge treatment at 700 rpm for 5 minutes. When the fluorescence intensity of the slide glass surface was measured by a fluorescence scanning device, it was 35.5 at the position where the antibody was spotted at 100 ng/ µL, indicating a large increase from a background fluorescence intensity. Therefore, it is understood that by employing the antibody-fixed solid support of the present invention where the antibody is bound via sulfonyl group, a ligand having reactivity with the antibody fixed to the antibody fixed-solid support can be efficiently detected.

### Example 4: Preparation of oligonucleotide-fixed solid support and measurement of amount of fixed oligonucleotide (Referential Example)

### (1) Preparation of gold electrode to the surface of which vinylsulfonyl group is fixed

To the surface of a gold electrode (surface area: 2.25mm²) which was washed with acetone, 2µL of 11-amino-1-undecathiol aqueous solution (1 mM) was dropped. Then, the gold electrode was left for 10 hours while the solution was not dried, and the surface of electrode was washed with distilled water and ethanol in turn. Then, 2 µL of phosphate buffer solution (pH8.5) containing 3% of 1,2-bis(vinylsulfonylacetamide)ethane was dropped onto the surface of the gold electrode, and the gold electrode was left for 2 hours at room temperature, and the surface was washed with distilled water and ethanol in turn. Then, the surface was dried for 1 hour under a reduced pressure to obtain a gold electrode to the surface of which a vinylsulfonyl group was bound via a linking group.

### (2) Fixation of oligonucleotide (Preparation of electrochemical analyzing element)

2 µL of an aqueous solution (100pM/1 µL) of an oligonucleotide of thymine 20mer (T20) of which 5' end was introduced with an aminohexyl group, was dropped to the gold electrode having a vinylsulfonyl group on the surface obtained in the above (1). Then, the gold electrode was left for 1 hour at room temperature, washed to remove the excess oligonucleotide (T20), and then dried to prepare an electrochemical analyzing element.

### (3) Preparation of ferrocene-labeled oligonucleotide

An aminohexyl group-bonded oligonucleotide was obtained by bonding an aminohexyl group linker to 5' end of 20 mer of adenine (A20).

Using the aminohexyl group-bonded oligonucleotide obtained above, an oligonucleotide of 20 mer of adenine whose 5' end was labeled with ferrocene (F1-A20) was prepared according to a method described in Analytical Biochemistry, Takenaka et al., 217: 436-443 (1994).

### (4) Detection of complementary target oligonucleotide sample

2 *µ*L of 10mM Tris buffer solution (pH 7.5) containing the oligonucleotide of 20 mer of adenine whose 5' end was labeled with ferrocene (F1-A20) mentioned above was dropped to the surface of the electrochemical analyzing element prepared in the above (2). Then, the electrochemical analyzing element was incubated at 25°C for 30 minutes. After the completion of the incubation, the surface of the analyzing element was washed with pure water, and unreacted compound F1-A20 was removed.

When the differential pulse voltammetry (DVP) was performed in an applied voltage range from 100 to 700 mV utilizing a 0.1 M potassium chloride-0.1 M acetic acid buffer solution (pH 5.60) as a measurement solution (38°C), response current derived from the compound F1-A20 was obtained at 460 mV of the applied voltage.

It has been confirmed by the above-mentioned procedures and results that an oligonucleotide has been stably bonded and fixed to the surface of the gold electrode, and that using this electrode, a complementary target nucleotide sample labeled with an electrochemical labeling substance can be detected.

According to the present invention, it has become possible to provide a biological material chip prepared by bonding and fixing at least one member of specific bonding partners to a reactive solid support which can achieve rapid and stable binding and fixing.

## Claims

1. A biological material chip wherein a group represented by following formula (I) which contains a residue of a member of specific binding partners is bound to a solid support, wherein the formula is
-L-SO₂-X-A (I)
and in the formula (I), L represents a linking group which binds -SO₂-X-A and the solid support; X represents -CR¹(R²)-CR³(R⁴)-; each of R¹, R², R³ and R⁴ represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and A represents a residue of the member of specific binding partners, said specific binding partners being a combination of an antibody or its fragment and a ligand, a combination of an antibody or its fragment and an antigen, a combination of an antibody or its fragment and a hapten, a combination of avidins and biotins or a combination of a receptor and a ligand and said solid support being glass, an electrode surface or a sensor chip surface.

2. The biological material chip as claimed in claim 1, wherein said avidins are avidin, streptavidin, or their altered bodies which can form a stable complex with biotin.

3. The biological material chip as claimed in claim 1, wherein said biotins are biotin, biocytin, desthiobiotin, oxybiotin, or their derivatives which can form a stable complex with avidin.

4. The biological material chip as claimed in claim 1, wherein said A represents a residue of a protein in the formula (I).

5. A method for the detection of a target substance in a specimen comprising the steps of:
(a) contacting a biological material chip wherein a group represented by following formula (I) containing a residue of a member of specific binding partners is bound to a solid support with a specimen containing a target substance which is another member of the specific binding partners; and
(b) analyzing interaction between said members of specific binding partners, wherein the formula is
-L-SO₂-X-A (I)
and in the formula (I), L represents a linking group which binds -SO₂-X-A and the solid support; X represents -CR¹(R²)-CR³(R⁴)-; each of R¹, R², R³ and R⁴ represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and A represents a residue of one member of specific binding partners, said specific binding partners being a combination of an antibody or its fragment and a ligand, a combination of an antibody or its fragment and an antigen, a combination of an antibody or its fragment and a hapten, a combination of avidins and biotins or a combination of a receptor and a ligand and said solid support being glass, an electrode surface or a sensor chip surface.

6. The method for the detection of a target substance as claimed in claim 5, wherein said avidins are avidin, streptavidin, or their altered bodies which can form a stable complex with biotin.

7. The method for the detection of a target substance as claimed in claim 5, wherein said biotins are biotin, biocytin, desthiobiotin, oxybiotin, or their derivatives which can form a stable complex with avidin.

8. The method for the detection of a target substance as claimed in claim 5, wherein said A represents a residue of a protein in the formula (I).

9. The method for the detection of a target substance as claimed in claim 5, wherein a free reactive group that exists on a surface of a solid support to which a group represented by the formula (I) containing a residue of a member of specific binding partners is bound, is subjected to a blocking treatment with an aqueous solution of an amino acid, a peptide or a protein.

10. A method for the production of a biological material chip as claimed in claim 1, comprising a step of contacting at least one member of specific binding partners containing a reactive group which forms a covalent bond by reacting with a vinylsulfonyl group or its reactive precursor group represented by following formula (II), with a solid support having the vinylsulfonyl group or its reactive precursor group represented by following formula (II) on its surface, wherein the formula is
-L-SO₂-X' (II)
and in the formula (II), L represents a linking group which binds -SO₂-X' and the solid support; X' represents -CR¹=CR² (R³) or -CH(R¹)-CR²(R³) (Y) ; each of R¹, R² and R³ represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; Y-represents a group which is substituted by a nucleophilic reagent or a group which is eliminated as [HA] by a base, said specific binding partners being a combination of an antibody or its fragment and a ligand, a combination of an antibody or its fragment and an antigen, a combination of an antibody or its fragment and a hapten, a combination of avidins and biotins or a combination of a receptor and a ligand and said solid support being glass, an electrode surface or a sensor chip surface.

11. The method for the production of a biological material chip as claimed in claim 10, wherein said avidins are avidin, streptavidin, or their altered bodies which can form a stable complex with biotin.

12. The method for the production of a biological material chip as claimed in claim 10, wherein said biotins are biotin, biocytin, desthiobiotin, oxybiotin, or their derivatives which can form a stable complex with avidin.

13. The method for the production of a biological material chip as claimed in claim 10, wherein said member of specific binding partners to be contacted with said solid support is a protein.

14. The method for the production of a biological material chip as claimed in claim 10, comprising a step of performing a blocking treatment of a free reactive group on surface of said solid support with an aqueous solution of an amino acid, a peptide or a protein, after contacting at least one member of specific binding partners with said solid support.

## Patentansprüche

1. Chip aus biologischem Material, wobei eine Gruppe, dargestellt durch die folgende Formel (I), die einen Rest eines Mitglieds spezifischer Bindungspartner enthält, an einen festen Träger gebunden ist, wobei die Formel
-L-SO₂-X-A (I)
ist, und in der Formel(I), L eine Verknüpfungsgruppe darstellt, die -SO₂-X-A und den festen Träger verbindet; X -CR¹(R²)-CR³(R⁴)- darstellt; jedes von R¹, R², R³ und R⁴ unabhängig von einander ein Wasserstoffatom, eine Alkylgruppe mit 1 bis 6 Kohlenstoffatomen, eine Arylgruppe mit 6 bis 20 Kohlenstoffatomen oder eine Aralkylgruppe mit insgesamt 7 bis 26 Kohlenstoffatomen, die eine Alkylkette mit 1 bis 6 Kohlenstoffatomen enthält, darstellt; und A einen Rest des Mitglieds der spezifischen Bindungspartner darstellt, wobei die spezifischen Bindungspartner eine Kombination eines Antikörpers oder seines Fragments und eines Liganden, eine Kombination eines Antikörpers oder seines Fragments und eines Antigens, eine Kombination eines Antikörpers oder seines Fragments und eines Haptens, eine Kombination von Avidinen und Biotinen oder eine Kombination eines Rezeptors und eines Liganden sind, und der feste Träger Glas, eine Elektrodenoberfläche oder eine Sensorchipoberfläche ist.

2. Chip aus biologischem Material nach Anspruch 1, wobei die Avidine Avidin, Streptavidin oder ihre veränderten Körper sind, die einen stabilen Komplex mit Biotin bilden können.

3. Chip aus biologischem Material nach Anspruch 1, wobei die Biotine Biotin, Biocytin, Desthiobiotin, Oxybiotin oder deren Derivate sind, die einen stabilen Komplex mit Avidin bilden können.

4. Chip aus biologischem Material nach Anspruch 1, wobei A einen Rest eines Proteins in der Formel (I) darstellt.

5. Verfahren zur Detektion einer Zielsubstanz in einer Probe, umfassend die Schritte:
(a) In-Kontakt-Bringen eines Chips aus biologischem Material, wobei eine Gruppe, dargestellt durch die folgende Formel (I), die einen Rest eines Mitglieds der spezifischen Bindungspartner enthält, an einen festen Träger gebunden ist, mit einer Probe, die eine Zielsubstanz enthält, die ein anderes Mitglied der spezifischen Bindungspartner ist; und
(b) Analysieren der Wechselwirkung zwischen den Mitgliedern der spezifischen Bindungspartner, wobei die Formel
-L-SO₂-X-A (I)
ist, und in der Formel(I), L eine Verknüpfungsgruppe darstellt, die -SO₂-X-A und den festen Träger verbindet; X -CR¹(R²)-CR³(R⁴)- darstellt; jedes von R¹, R², R³ und R⁴ unabhängig von einander ein Wasserstoffatom, eine Alkylgruppe mit 1 bis 6 Kohlenstoffatomen, eine Arylgruppe mit 6 bis 20 Kohlenstoffatomen oder eine Aralkylgruppe mit insgesamt 7 bis 26 Kohlenstoffatomen, die eine Alkylkette mit 1 bis 6 Kohlenstoffatomen enthält, darstellt; und A einen Rest eines Mitglieds der spezifischen Bindungspartner darstellt, wobei die spezifischen Bindungspartner eine Kombination eines Antikörpers oder seines Fragments und eines Liganden, eine Kombination eines Antikörpers oder seines Fragments und eines Antigens, eine Kombination eines Antikörpers oder seines Fragments und eines Haptens, eine Kombination von Avidinen und Biotinen oder eine Kombination eines Rezeptors und eines Liganden sind, und der feste Träger Glas, eine Elektrodenoberfläche oder eine Sensorchipoberfläche ist.

6. Verfahren zur Detektion einer Zielsubstanz nach Anspruch 5, wobei die Avidine Avidin, Streptavidin oder ihre veränderten Körper sind, die einen stabilen Komplex mit Biotin bilden können.

7. Verfahren zur Detektion einer Zielsubstanz nach Anspruch 5, wobei die Biotine Biotin, Biocytin, Desthiobiotin, Oxybiotin oder deren Derivate sind, die einen stabilen Komplex mit Avidin bilden können.

8. Verfahren zur Detektion einer Zielsubstanz nach Anspruch 5, wobei A einen Rest eines Proteins in der Formel (I) darstellt.

9. Verfahren zur Detektion einer Zielsubstanz nach Anspruch 5, wobei eine freie reaktive Gruppe, die auf einer Oberfläche eines festen Trägers vorhanden ist, an den eine Gruppe, dargestellt durch die Formel (I), die einen Rest eines Mitglieds der spezifischen Bindungspartner enthält, gebunden ist, einer Blockierungsbehandlung mit einer wässrigen Lösung einer Aminosäure, eines Peptids oder eines Proteins unterzogen wird.

10. Verfahren zur Herstellung eines Chips aus biologischem Material nach Anspruch 1, umfassend einen Schritt des In-Kontakt-Bringens von mindestens einem Mitglied der spezifischen Bindungspartner, das eine reaktive Gruppe enthält, die eine kovalente Bindung durch Reagieren mit einer Vinylsulfongruppe oder deren reaktiver Vorläufergruppe, dargestellt durch die folgende Formel (II), bildet, mit einem festen Träger, der eine Vinylsulfonylgruppe oder deren reaktive Vorläufergruppe, dargestellt durch die folgende Formel (II), auf seiner Oberfläche hat, wobei die Formel
-L-SO₂-X' (II)
ist, und in der Formel (II), L eine Verknüpfungsgruppe darstellt, die -SO₂-X' und den festen Träger verbindet; X' -CR¹=CR²(R³) oder -CH(R¹)-CR²(R³) (Y) darstellt; jedes von R¹, R² und R³ unabhängig von einander ein Wasserstoffatom, eine Alkylgruppe mit 1 bis 6 Kohlenstoffatomen, eine Arylgruppe mit 6 bis 20 Kohlenstoffatomen oder eine Aralkylgruppe mit insgesamt 7 bis 26 Kohlenstoffatomen, die eine Alkylkette mit 1 bis 6 Kohlenstoffatomen enthält, darstellt; Y eine Gruppe darstellt, die durch ein nukleophiles Reagens substituiert ist, oder eine Gruppe, die als [HA] durch eine Base eliminiert wird, wobei die spezifischen Bindungspartner eine Kombination eines Antikörpers oder seines Fragments und eines Liganden, eine Kombination eines Antikörpers oder seines Fragments und eines Antigens, eine Kombination eines Antikörpers oder seines Fragments und eines Haptens, eine Kombination von Avidinen und Biotinen oder eine Kombination eines Rezeptors und eines Liganden sind, und der feste Träger Glas, eine Elektrodenoberfläche oder eine Sensorchipoberfläche ist.

11. Verfahren zur Herstellung eines Chips aus biologischem Material nach Anspruch 10, wobei die Avidine Avidin, Streptavidin oder ihre veränderten Körper sind, die einen stabilen Komplex mit Biotin bilden können.

12. Verfahren zur Herstellung eines Chips aus biologischem Material nach Anspruch 10, wobei die Biotine Biotin, Biocytin, Desthiobiotin, Oxybiotin oder deren Derivate sind, die einen stabilen Komplex mit Avidin bilden können.

13. Verfahren zur Herstellung eines Chips aus biologischem Material nach Anspruch 10, wobei das Mitglied der spezifischen Bindungspartner, das mit dem festen Träger in Kontakt gebracht werden soll, ein Protein ist.

14. Verfahren zur Herstellung eines Chips aus biologischem Material nach Anspruch 10, umfassend einen Schritt des Durchführens einer Blockierungsbehandlung einer freien reaktiven Gruppe auf einer Oberfläche des festen Trägers mit einer wässrigen Lösung einer Aminosäure, eines Peptids oder eines Proteins nach dem In-Kontakt-Bringen von mindestens einem Mitglied der spezifischen Bindungspartner mit dem festen Träger.

## Revendications

1. Puce de matériaux biologiques dans laquelle un groupe représenté par la formule (I) ci-après qui contient un résidu d'un élément de partenaires de liaison spécifique est lié à un support solide, dans laquelle la formule est
-L-SO₂-X-A (I)
et dans la formule (I), L représente un groupe de liaison qui lie -SO₂-X-A et le support solide ; X représente -CR¹(R²)-CR³(R⁴)-; R¹, R², R³ et R⁴ représentent chacun indépendamment les uns des autres un atome d'hydrogène, un groupe alkyle possédant 1 à 6 atomes de carbone, un groupe aryle possédant 6 à 20 atomes de carbone, ou un groupe aralkyle possédant 7 à 26 atomes de carbone contenant en tout une chaîne alkyle possédant 1 à 6 atomes de carbone ; et A représente un résidu de l'élément de partenaires de liaison spécifique, lesdits partenaires de liaison spécifique étant une combinaison d'un anticorps ou son fragment et d'un ligand, une combinaison d'un anticorps ou son fragment et d'un antigène, une combinaison d'un anticorps ou son fragment et d'un haptène, une combinaison d'avidines et de biotines ou une combinaison d'un récepteur et d'un ligand et ledit support solide étant du verre, une surface d'électrode ou une surface de biocapteur.

2. Puce de matériaux biologiques selon la revendication 1, dans laquelle lesdites avidines sont l'avidine, la streptavidine, ou leurs corps modifiés qui peuvent former un complexe stable avec la biotine.

3. Puce de matériaux biologiques selon la revendication 1, dans laquelle lesdites biotines sont la biotine, la biocytine, la desthiobiotine, l'oxybiotine, ou leurs dérivés qui peuvent former un complexe stable avec l'avidine.

4. Puce de matériaux biologiques selon la revendication 1, dans laquelle ledit A représente un résidu d'une protéine dans la formule (I).

5. Procédé de détection d'une substance cible dans un échantillon comprenant les étapes consistant à :
(a) mettre en contact une puce de matériaux biologiques dans laquelle un groupe représenté par la formule (I) ci-après contenant un résidu d'un élément de partenaires de liaison spécifique est lié à un support solide avec un échantillon contenant une substance cible qui est un autre élément des partenaires de liaison spécifique ; et
(b) analyser l'interaction entre lesdits éléments de partenaires de liaison spécifique, dans lequel la formule est
-L-SO₂-X-A (I)
et dans la formule (I), L représente un groupe de liaison qui lie -SO₂-X-A et le support solide ; X représente -CR¹(R²)-CR³(R⁴)-; R¹, R² R³ et R⁴ représentent chacun indépendamment les uns des autres un atome d'hydrogène, un groupe alkyle possédant 1 à 6 atomes de carbone, un groupe aryle possédant 6 à 20 atomes de carbone, ou un groupe aralkyle possédant 7 à 26 atomes de carbone contenant en tout une chaîne alkyle possédant 1 à 6 atomes de carbone ; et A représente un résidu d'un élément de partenaires de liaison spécifique, lesdits partenaires de liaison spécifique étant une combinaison d'un anticorps ou son fragment et d'un ligand, une combinaison d'un anticorps ou son fragment et d'un antigène, une combinaison d'un anticorps ou son fragment et d'un haptène, une combinaison d'avidines et de biotines ou une combinaison d'un récepteur et d'un ligand et ledit support solide étant du verre, une surface d'électrode ou une surface de biocapteur.

6. Procédé de détection d'une substance cible selon la revendication 5, dans lequel lesdites avidines sont l'avidine, la streptavidine, ou leurs corps modifiés qui peuvent former un complexe stable avec la biotine.

7. Procédé de détection d'une substance cible selon la revendication 5, dans lequel lesdites biotines sont la biotine, la biocytine, la desthiobiotine, l'oxybiotine, ou leurs dérivés qui peuvent former un complexe stable avec l'avidine.

8. Procédé de détection d'une substance cible selon la revendication 5, dans lequel ledit A représente un résidu d'une protéine dans la formule (I).

9. Procédé de détection d'une substance cible selon la revendication 5, dans lequel un groupe réactif libre qui existe sur une surface d'un support solide à laquelle un groupe représenté par la formule (I) contenant un résidu d'un élément de partenaires de liaison spécifique est lié, est soumis à un traitement de blocage avec une solution aqueuse d'un acide aminé, d'un peptide ou d'une protéine.

10. Procédé de production d'une puce de matériaux biologiques selon la revendication 1, comprenant une étape consistant à mettre en contact au moins un élément de partenaires de liaison spécifique contenant un groupe réactif qui forme une liaison covalente en réagissant avec un groupe vinylsulfonyle ou son groupe réactif précurseur représenté par la formule (II) ci-après, avec un support solide possédant le groupe vinylsulfonyle ou son groupe réactif précurseur représenté par la formule (II) ci-après sur sa surface, dans lequel la formule est
-L-SO₂-X' (II)
et dans la formule (II), L représente un groupe de liaison qui lie -SO₂-X' et le support solide ; X' représente -CR¹=CR²(R³) ou CH(R¹)-CR²(R³) (Y) ; R¹, R² et R³ représentent chacun indépendamment les uns des autres un atome d'hydrogène, un groupe alkyle possédant 1 à 6 atomes de carbone, un groupe aryle possédant 6 à 20 atomes de carbone, ou un groupe aralkyle possédant 7 à 26 atomes de carbone contenant en tout une chaîne alkyle possédant 1 à 6 atomes de carbone ; Y représente un groupe qui est remplacé par un réactif nucléophile ou un groupe qui est éliminé sous la forme [HA] par une base, lesdits partenaires de liaison spécifique étant une combinaison d'un anticorps ou son fragment et d'un ligand, une combinaison d'un anticorps ou son fragment et d'un antigène, une combinaison d'un anticorps ou son fragment et d'un haptène, une combinaison d'avidines et de biotines ou une combinaison d'un récepteur et d'un ligand et ledit support solide étant du verre, une surface d'électrode ou une surface de biocapteur.

11. Procédé de production d'une puce de matériaux biologiques selon la revendication 10, dans lequel lesdites avidines sont l'avidine, la streptavidine, ou leurs corps modifiés qui peuvent former un complexe stable avec la biotine.

12. Procédé de production d'une puce de matériaux biologiques selon la revendication 10, dans lequel lesdites biotines sont la biotine, la biocytine, la desthiobiotine, l'oxybiotine, ou leurs dérivés qui peuvent former un complexe stable avec l'avidine.

13. Procédé de production d'une puce de matériaux biologiques selon la revendication 10, dans lequel ledit élément de partenaires de liaison spécifique à mettre en contact avec ledit support solide est une protéine.

14. Procédé de production d'une puce de matériaux biologiques selon la revendication 10, comprenant une étape consistant à effectuer un traitement de blocage d'un groupe réactif libre en surface dudit support solide avec une solution aqueuse d'un acide aminé, d'un peptide ou d'une protéine, après avoir mis en contact au moins un élément de partenaires de liaison spécifique avec ledit support solide.


### SEQUENCE LISTING

<110> FUJI PHOTO FILM CO., LTD.
<120> Biological material chip
<130> 12284EP
<140>
   <141>
<150> JP2001-138496
   <151> 2001-05-09
<160> 7
<170> PatentIn Ver. 2.1
<210> 1
   <211> 40
   <212> DNA
   <213> Artificial Sequence
<220>
   <223> Description of Artificial Sequence:oligonucleotide fragment
<400> 1
   tcctccatgt ccggggagga tctgacactt caaggtctag 40
<210> 2
   <211> 22
   <212> DNA
   <213> Artificial Sequence
<220>
   <223> Description of Artificial Sequence:oligonucleotide fragment
<400> 2
   ctagtctgtg aagttccaga tc 22
<210> 3
   <211> 20
   <212> DNA
   <213> Artificial Sequence
<220>
   <223> Description of Artificial Sequence: primer
<400> 3
   tggccgcctt caacgctcag 20
<210> 4
   <211> 24
   <212> DNA
   <213> Artificial Sequence
<220>
   <223> Description of Artificial Sequence:primer
<400> 4
   gaaggtgtgg cgcaggtcgt agtg 24
<210> 5
   <211> 23
   <212> DNA
   <213> Artificial Sequence
<220>
   <223> Description of Artificial Sequence:primer
<400> 5
   atggatgatg atatcgccgc gct 23
<210> 6
   <211> 24
   <212> DNA
   <213> Artificial Sequence
<220>
   <223> Description of Artificial Sequence:primer
<400> 6
   ggtgaggatc ttcatgaggt agtc 24
<210> 7
   <211> 22
   <212> DNA
   <213> Artificial Sequence
<220>
   <223> Description of Artificial Sequence:primer
<400> 7
   actgtgcgtg ttttccgggg gt 22